# EUROPÄISCHE PATENTSCHRIFT

(11) **EP 1 438 088 B1**
(45) Veröffentlichungstag und Bekanntmachung des Hinweises auf die Patenterteilung: **02.12.2009**
(21) Anmeldenummer: 02782926.6
(22) Anmeldetag: 16.10.2002
(51) Int. Cl.: A61M 1/02

(54) **Medizinisches Beutelsystem für Kryokonservierung**
Medical Bag system for Cryo-preservation
Système de sachets médicaux pour la cryo-conservation

(30) Priorität: 22.10.2001 DE 10151343
(43) Veröffentlichungstag der Anmeldung: 21.07.2004
(73) Patentinhaber: Lampeter, Dr. med., Eberhard, 04299 Leipzig (DE); Egger, Dr. rer. Nat., Dietmar, 72116 Mössingen (DE)
(72) Erfinder: Lampeter, Dr. med., Eberhard, 04299 Leipzig (DE); Egger, Dr. rer. Nat., Dietmar, 72116 Mössingen (DE)
(74) Vertreter: Müller, Volkmar
(86) Internationale Anmeldenummer: PCT/EP2002/011564
(87) Internationale Veröffentlichungsnummer: WO 2003/041634

(56) Entgegenhaltungen:
- EP-A- 0 542 221
- US-A- 5 356 373
- US-A- 5 879 318
- US-A- 6 059 968
- US-B1- 6 232 115

## Beschreibung

Die Erfindung betrifft ein Beutelsystem für die Kryokonservierung von Körperflüssigkeiten, nämlich Blut, Knochenmark oder Nabelschnurblut, wobei die Teile des Beutelsystems sterilisiert und voneinander abtrennbar sind und sich Flüssigkeiten, die eine Kryokonservierung ermöglichen und/oder unterstützen, im Beutelsystem befinden und/oder sich steril in das Beutelsystem eintragen lassen, wie im Anspruch 1 definiert.

Die Erfindung kann benutzt werden in einem Verfahren zur Bereitstellung von Körperflüssigkeiten, nämlich Blut, Knochenmark oder Nabelschnurblut, zur Langzeitlagerung durch Kryokonservierung in einem Transport- und Lagersystem, zumindest umfassend die Schritte: Einbringen der Körperflüssigkeit ins Transport- und Lagersystem, Vorlegen oder Zugabe von Flüssigkeiten, die die Kryokonservierung ermöglichen und/oder unterstützen, sowie überführen des Gemisches aus Flüssigkeiten, die die Kryokonservierung ermöglichen und/oder unterstützen, und der Körperflüssigkeit in einen Bereich zur Einlagerung im Transport- und Lagersystem.

Die Kryokonservierung von Körperflüssigkeiten, nämlich Blut, Knochenmark oder Nabelschnurblut, hat einhergehend mit der stetigen Entwicklung der Humanmedizin eine immense wirtschaftliche Bedeutung erlangt.
Nabelschnurblut und dessen Konservierung über mehrere Jahre, sog. Langzeitkonservierung, sind im Zusammenhang mit einer eventuellen späteren Behandlung des Kindes, des Jugendlichen oder des Erwachsenen mit Mitteln, die auf körpereigenen Stoffen basieren, z.b. im Rahmen einer Transplantation, von entscheidender Bedeutung. Dieses wertvolle Blut steht nämlich nur bei der Geburt des Kindes zur Verfügung. Nach der Abnabelung ist in der Nabelschnur noch Blut vorhanden, das für die Kindsversorgung nicht benötigt wird. Dieses Blut, regelmäßig ein Blutmenge mit einem Volumen von ca. 50 bis 100 ml, wird, in dem Fall, wo eine Langzeitkonservierung vorgesehen ist, aufgenommen und für die Kryokonservierung aufbereitet und nachfolgend durch bekannte Verfahren eingefroren. Die vorgenannten geringen Mengen des entnommenen Blutes müssen zweckmäßigerweise in einer Art und Weise eingefroren und gelagert werden, dass mehrere Proben unabhängig und zeitlich voneinander, ggf. im Abstand von mehreren Jahren, nach den dann verfügbaren neuesten Methoden untersucht und eingesetzt werden Können. In Ermangelung heutiger Kenntnis der dann angewandten Methoden ist die Kryokonservierung aller Bestandteile der entnommenen Körperflüssigkeit angezeigt.
Derzeit werden für die Aufbereitung und das Einfrieren getrennte Systeme verwendet. So erfolgt die Aufnahme des Nabelschnurblutes durch ein System, welches regelmäßig besteht aus einer oder mehreren Abnahmekanülen, einem oder zwei Behältnissen, die Zitrat enthalten, sowie dem eigentlichen Blut-Sammelbehälter, wobei die vorgenannten Teile des Systems durch flexible Schläuche miteinander kommunizierend verbunden sind. Ein solches System ist beispielsweise in der US 5,879,318 oder EP-A-542 221 als Stand der Technik genannt.

Nach der Aufbereitung des Blutes erfolgt nachfolgend das Umfüllen von einem System in ein anderes zum Zwecke der Kryokonservierung, also von einem sterilen Behälter in einen sterilen Einfrierbehälter, was regelmäßig unter Reinstraumbedingungen erfolgen muß. Die Gewährleistung der gesetzlich vorbestimmten Reinstraumbedingungen bezüglich der Humanmedizin, beispielsweise die Anforderungen gemäß GMP, erfordern regelmäßig einen hohen Investitionsaufwand, der üblicherweise nicht in jeder Geburtenstation oder medizinischen Einrichtung, die Körperflüssigkeiten steril entnimmt und für die Kryokonservierung vorbereiten, realisiert werden kann.

Bisher erfolgt die Vorbereitung der Kryokonservierung von Nabelschnurblut grundsätzlich in folgenden Schritten:

Entnahme des Blutes und Einbringung in den Transportbeutel. Zugabe von Zitrat, soweit nicht schon vorgelegt, Verschließen des Beutels und Transport bei Zimmertemperatur zu einem Reinstraum innerhalb von ca. 24 bis 48 Stunden. Überführen des Blutes und Zugabe eines Kryoprotektivums aus dem Transportbeutel in einen sterilen Einfrierbehälter unter Reinstraumbedingungen, wobei immer zumindest zwei getrennte Systeme zur Anwendung kommen.

Aufgabe der Erfindung ist es, ein Beutelsystem bereitzustellen, das die Kosten der Vorbereitung und Durchführung der Kryokonservierung reduziert und eine einfache und sichere manuelle Handhabung erlaubt, wobei die Anforderungen zur Sterilität in der Humanmedizin zu gewährleisten sind. Außerdem kann die Erfindung, in ein Verfahren zur Bereitstellung von Körperflüssigkeiten, nämlich von Blut, Knochenmark oder Nabelschnurblut benutzt werden zur Langzeitlagerung durch Kryokonservierung, welches ein Transport- und Lagersystem der vorgenannten Art einsetzt.

Die Aufgabe der Erfindung wird durch ein Beutelsystem gemäß den. Merkmalen des Anspruchs 1 gelöst.

Erfindungswesentlich ist, dass das Beutelsystem für die Kryokonservierung von Körperflüssigkeiten, nämlich Blut, Knochenmark oder Nabelschnurblut bestimmt ist, wobei die Teile des Beutelsystems sterilisiert, voneinander abtrennbar sind und sich Flüssigkeiten, die eine Kryokonservierung ermöglichen und/oder unterstützen, im Beutelsystem befinden und sich steril in das Beutelsystem eintragen lassen, zumindest umfassend:
- eine Vorrichtung zur direkten Entnahme der Körperflüssigkeit (1) aus dem lebenden Körper,
- zumindest einen Bereich zum Mischen der Flüssigkeiten mit der Körperflüssigkeit und zumindest einen Bereich zur Einlagerung des Gemisches aus Flüssigkeiten und Körperflüssigkeit, nämlich einen Mischbeutel und einen Einfrierbeutel, wobei dieser Einfrierbeutel eine Einfrierkammer oder zumindest zwei Einfrierkammern besitzt;
- Verbindungsleitungen und mehrere Absperrorgane zum Absperren jeweils einer der Verbindungsleitungen, wobei die Teile des Beutelsystems, die durch die Verbindungsleitungen miteinander kommunizierend verbunden sind, vor dem Einsatz des Beutelsystems ein geschlossenes, steriles System bilden, und dass die Bereiche zur Einlagerung des Gemisches aus Flüssigkeiten und der Körperflüssigkeit voneinander hermetisch verschließbar und abtrennbar sind,
wobei das Beutelsystem ein Einlaßorgan für Flüssigkeiten, welches ein Steril-Filter ist, umfasst, und der Mischbeutel und der Einfrierbeutel durch zwei Verbindungsleitungen miteinander verbunden sind, wobei ein geschlossener Kreislauf zwischen den beiden Beuteln besteht.

Die Aufgabe der Erfindung wird dadurch gelöst, dass die Teile des Beutelsystems, die durch die Verbindungsleitungen miteinander kommunizierend verbunden sind, vor dem Einsatz des Beutelsystems ein geschlossenes, steriles System bilden und die Bereiche zur Einlagerung des Gemisches aus Flüssigkeiten und der Körperflüssigkeit voneinander hermetisch verschließbar und abtrennbar sind.
Die Erfindung ermöglicht es, ein Beutelsystem bereitzustellen, welches zumindest zum Zeitpunkt des Einbringens der Körperflüssigkeit, insbesondere direkt aus dem menschlichen Körper, ein sog, geschlossenes System bildet. Damit wird bezüglich der Sterilität ein Optimun erzielt und es besteht die Möglichkeit, Teile des Beutelsystems, die insbesondere im Zusammenhang mit der eigentlichen Kryokonservierung nicht mehr benötigt werden, schrittweise vom Beutelsystem zu trennen.
Vom Beutelsystem abgetrennt werden die Teile, die für das zweckdienliche Handling nicht mehr benötigt werden, wie beispielsweise die Vorrichtung zur Aufnahme der Körperflüssigkeit nach der Überführung der Körperflüssigkeit in einen Bereich zum Mischen. Zum Zeitpunkt des Einfrierens besteht das geschlossene System dann nur noch aus voneinander getrennten und hermetisch verschlossenen Teilen, die nicht mehr miteinander kommunizierend verbunden sind. Einsparungen bezüglich von Transport- und Lagertuigskosten werden damit erzielt.
Durch das erfinderische Merkmal, dass die Bereiche zur Einlagerung des Gemisches aus Flüssigkeiten, die die Kryokonservierung ermöglichen und/oder unterstützen, und der überführten Körperflüssigkeit voneinander hermetisch verschließbar und abtrennbar sind, wird es möglich, die Gesamtmenge des vorgenannten Gemisches in mehrere voneinander getrennte und bezüglich der Größe ihres Volumens in gewünschte Teilmengen zu separieren. Diese separierten Teilmengen stehen insbesondere nach dem Einfrieren ohne Eröffnung des gesamten Beutelsystems für bakteriologische und serologische Untersuchungen und zur Überwachung der Dauerlagerung zur Verfügung. Das gesamte Handling im Labor, beispielsweise die Zugabe eines Kryoprotektivum über einen Sterilfilter, kann unter normalen hygienischen Bedingungen (beispielsweise Reinraum Klasse D) erfolgen; Reinstraumbedingungen sind nicht erforderlich.

Die eingesetzten Materialien für die Teile des Beutelsystems sind in bekannter Art und Weise, insbesondere auf die Funktion des jeweiligen Teiles, der Art des oder der Medien mit denen es in Berührung kommt, die Einwirkzeit des Mediums, die Art und Weise der Sterilisation sowie dem Temperaturregime, d.h. insbesondere der größten und kleinsten während des Handlings und Einfrierens anzutreffenden Temperatur, ausgewählt und aufeinander angepasst. Bekannte Kunststoffe, die die vorgenannten Anforderungen erfüllen und für Anwendungen in der Humanmedizin zugelassen sind, stehen dabei im Fokus.

Flüssigkeiten, die die Kryokonservierung ermöglichen und/oder unterstützen, sind im Sinne der Erfindung diesbezüglich bekannte Medien. Für die Kryokonservierung von Blut sind das beispielsweise flüssige Mittel zur Verhinderung der Blutgerinnung, wie Citrat Phosphat Dextrose (CPD), zur Verdünnung des Blutes, wie Kochsalzlösungen, und Kryoprotektiva, wie DMSO.

Bereiche zum Mischen der Flüssigkeiten mit der Körperflüssigkeit und/oder zur Einlagerung sind im Sinne der Erfindung alle bekannten verschließbaren Flüssigkeitsspeicher, wie beispielsweise Behälter und Beutel der Medizin- und Labortechnik, die zur Atmosphäre hin flüssigkeitsdicht ausgebildet sind.

In einer bevorzugten erfindungsgemäßen Ausgestaltung sind die Bereiche zur Einlagerung des Gemisches aus Flüssigkeiten, und der Körperflüssigkeit derart gestaltet, dass diese zumindest zwei Kammern eines Beutels sind. Zwei Kammern ermöglichen die Separation von zwei Teilmengen des einzulagernden Flüssigkeitsgemisches, so dass eine bezüglich der gesamten Flüssigkeitsmenge praktisch auch zweckmäßige Aufteilung möglich ist. Die Verwendung eines Beutels, der flexible Wände aufweist, ist besonders vorteilhaft, wenn das Handling manuell durch Hebammen oder medizinisches Personal realisiert wird. Manuelle Druckeinwirkung auf die flexiblen Wände und die Ausnutzung der Schwerkraft bewirken den Flüssigkeits- und Gastransport innerhalb des Beutelsystems und ein inniges Durchmischen.

Eine weitere vorteilhafte Ausgestaltung ist, dass ein Bereich zur Einlagerung des Gemisches aus Flüssigkeiten und der Körperflüssigkeit eine Verbindungsleitung ist, die vorzugsweise mehrere voneinander hermetisch verschließbare und abtrennbare Segmente besitzt. Durch diese Gestaltung wird erreicht, dass in einfacher Art und Weise, beispielsweise durch hermetisches Verschließen durch Verschweißen der flexiblen und transparenten Verbindungsleitung, die zwischen dem Einfrierbeutel und dem Mischbeutel oder zwischen dem Einfrierbeutel und dem zur Entlüftung genutzten Sterilfilter angeordnet ist, kleinere Teilmengen separiert werden, die insbesondere als Proben zur Kontrolle der Langzeitkonservierung dienen.

Vorteilhaft ist außerdem, dass zumindest im Jeweiligen Bereich zum Mischen der Flüssigkeiten eine Füllstandsanzeige angeordnet ist. Die Füllstandsanzeige kann durch Aufbringen entsprechender Markierungen auf der transparenten Beutelwand erfolgen, so dass insbesondere die Mindestfüllhöhe für die eingebrachte Körperflüssigkeit und die Soll-Füllhöhe, die nach dem Auffüllen mit der Verdünnungsflüssigkeit erreicht sein soll, kontrolliert werden können.

Im Zusammenhang mit der Langzeitkonservierung ist es auch vorteilhaft, dass die von den anderen Teilen des Systems abgetrennten Bereiche zur Einlagerung des Gemisches aus Flüssigkeiten und der Körperflüssigkeit durch eine Umhüllung 8 umschlossen werden. Diese Umhüllung dient insbesondere dem mechanischen Schutz und kann zusätzlich ein gas- und flüssigkeitsdichtes Umhüllen gewährleisten.

Besonders vorteilhaft ist, dass das Beutelsystem, insbesondere hinsichtlich seiner Dimensionierung und der Materialauswahl, zur einmaligen Nutzung bestimmt ist. Sogenannte Ein-Weg-Systeme oder deren Teile besitzen eine Reihe von bekannten Vorteilen, wobei das Wissen über die nur einmalige Nutzung, ggf. nur kurze Verwendung, wie beispielsweise der Vorrichtung zur Entnahme der Körperflüssigkeit, eine preiswerte Herstellung des Teiles oder Systems ermöglichen.

Weniger Teile des Beutelsystems sind kostengünstiger und ermöglichen ein einfacheres Handlung. Dies ist beispielsweise dann der Fall, wenn gesichert ist, dass relativ kurzfristig, d.h. innerhalb von 10 bis 20 Minuten, nach der Aufnahme der Körperflüssigkeit ins Beutelsystem das Einfrieren beginnt.

Alternativ ist folgende Ausgestaltung des erfindungsgemäßen Beutelsystems bevorzugt, nämlich dass der Bereich zum Mischen der Flüssigkeiten mit der Körperflüssigkeit in einem Mischbeutel 11 und der Bereich zur Einlagerung des Gemisches aus Flüssigkeiten und Körperflüssigkeit in einem Einfrierbeutel 12 angeordnet sind, wobei dieser Beutel eine Einfrier-Kammer 9 oder zwei Einfrier-Kammem 9 besitzt.

Die Verwendung mehrerer Beutel, nämlich einem zum Mischen und einem zum Einlagern, die aus unterschiedlichen Materialien bestehen können, bewirkt ein verbessertes Handling und somit eine erhöhte Funktionssicherheit durch Ausschalten von subjektiven Manipulationsfehlern. Durch eine gezielte Materialauswahl ist es bei einem Beutelsystem für Nabelschnurblut beispielsweise möglich, im Mischbeutel CPD schon im Zusammenhang mit der Montage des gesamten Beutelsystems vorzulegen, so dass ein sonst steriles Einfüllen von CPD in das Beutelsystem unmittelbar vor der Aufnahme von Nabelschnurblut im Kreissaal entfällt. Außerdem ist es somit möglich, dass das Einfrieren erst innerhalb von ca. 20 Stunden nach der Aufnahme des Blutes ins Beutelsystem erfolgen muß, ohne das es zu signifikanten Schädigungen von Blutbestandteilen kommt, wobei bis zum Einfrieren eine Zwischenlagerung bei Zimmertemperatur realisierbar ist. Die Auswahl des Materials für diesen Einfrierbeutel 12 wird primär von seiner Temperaturbeständigkeit bezüglich der eigentlichen Kryokonservierung (Temperaturen bis ca. - 196 °C) bestimmt; eine Sterilisation durch Autoklavierung ist damit regelmäßig ausgeschlossen.

Für die vorgenannte Ausbildung des Beutelsystems mit zwei Beuteln zum Mischen und Einlagern sind der Mischbeutel 11 und der Einfrierbeutel 12 durch zwei Verbindungsleitungen miteinander verbunden. Blut bzw. Nabelschnurblut neigt, insbesondere beim Mischen mit anderen Flüssigkeiten zum Schäumen, d.h. in der Flüssigkeit bzw. im Flüssigkeitsgemisch sind Luftbläschen eingeschlossen, so dass ein Flüssigkeits-Luft-Gemisch vorliegt. Mit dieser Art der Anordnung, d.h. ein geschlossener Kreislauf zwischen den beiden Beuteln, kann das im Beutelsystem enthaltene Flüssigkeits-Luft-Gemisch in der Art manipuliert werden, das in der Flüssigkeitsmenge, die sich in dem Bereich oder den Bereichen zur Einlagerung befindet oder befinden, sich keine oder wenige Lufteinschlüsse in der Flüssigkeit zum Zeitpunkt des Einfrierens befinden.

Das Verfahren kann alternativ für die menschlichen Körperflüssigkeiten: Blut, Knochenmark oder Nabelschnurblut eingesetzt werden. Der Einsatz des Verfahrens ist grundsätzlich auch für andere Körperflüssigkeiten von Mensch und Tier geeignet.

Besonders verfahrensökonomisch ist es, dass eine definierte Menge des Gemisches aus Flüssigkeiten, die die Kryokonservierung ermöglichen und/oder unterstützen, und der Körperflüssigkeit in einen Bereich zur Einlagerung überführt wird. Das Überführen und Einlagern einer bestimmten Flüssigkeitsmenge, beispielsweise von 160 ml, ermöglicht ein einfaches und somit kostengünstiges Einfrieren mit herkömmlichen. Geräten zur Kryokonservierung.
Es ist außerdem bevorzugt, dass das Gemisch aus Flüssigkeiten, die die Kryokonservierung ermöglichen und/oder unterstützen, und der Körperflüssigkeit in einen Bereich zur Einlagerung nahezu blasenfrei überführt wird. Blasenfreiheit ist ein wichtiges Kriterium für die Qualität und somit die zweckentsprechende Brauchbarkeit des einzulagernden Flüssigkeitsgemisches. Dies erfordert beispielsweise die Anordnung eines Auslassorgans und/oder der entsprechenden Verbindungsleitungen, so dass eine manuelle Manipulation erfolgen kann bis das Flüssigkeitsgemisch nahezu blasenfrei vorliegt.

Im folgenden soll die Erfindung anhand eines Ausführungsbeispiels eines Beutelsystems für Nabelschnurblut .näher erläutert werden. Es zeigen:
- Figur 1: ein Beutelsystem mit einem Misch- und Einfrier-Beutel,
- Figur 2: ein Beutelsystem mit einem Misch- und Einfrier-Beutel, der eine Mischund Einfrier-Kammer und eine Einfrier-Kammer besitzt,
- Figur 3: ein Beutelsystem mit einem Misch- und Einfrier-Beutel, der eine Misch- und Einfrier-Kammer sowie einen Kochsalzbeutel besitzt,
- Figur 4: ein Beutelsystem mit einem Misch- und Einfrier-Beutel, der Beutel für eine Flüssigkeit zur Vermeidung der Blutgerinnung und einen Kochsalzbeutel besitzt,
- Figur 5: ein Beutelsystem, welches einen Einfrierbeutel mit zwei Kammern und einen Mischbeutel und zwei Verbindungsleitungen zwischen dem Einfrierbeutel und dem Mischbeutel besitzt,
- Figur 6: ein Beutelsystem, welches einen Einfrierbeutel mit zwei Kammern und einen Mischbeutel und eine Verbindungsleitung zwischen dem Einfrierbeutel und dem Mischbeutel besitzt,
- Figur 7: ein Beutelsystem, welches einen Einfrierbeutel mit einer Kammer, einen Mischbeutel und zwei Verbindungsleitungen zwischen dem Einfrierbeutel und dem Mischbeutel besitzt,
- Figur 8: ein Beutelsystem mit einer Glasampulle in einem Schutzbeutel und
- Figur 9: Teile eines Beutelsystems vor dem Einfrieren.

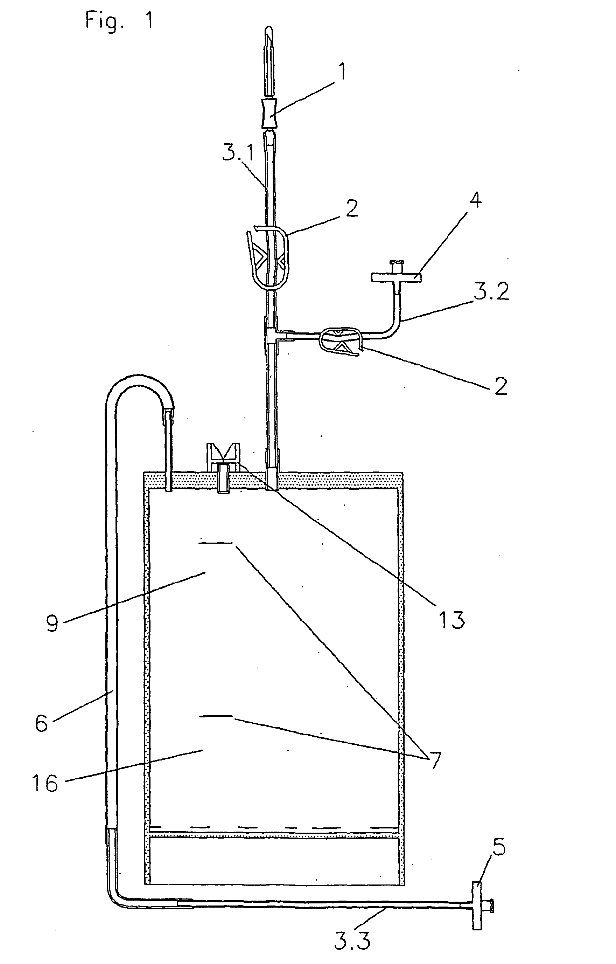

### (Die Figuren 1 bis 4 und 6 fallen nicht unter Anspruch 1).

Figur 1 zeigt ein Beutelsystem mit einem Misch- und Einfrier-Beutel 16, der durch drei Verbindungsleitungen 3,1, 3.2 und 3.3 mit einer Vorrichtung zur direkten Entnahme von Nabelschnurblut 1, einem Einlaßorgan 4 und einem Auslassorgan 5 kommunizierend verbunden ist. Die Kommunkation, d.h. insbesondere der Flüssigkeitstransport und der Luftaustausch, zwischen den vorgenannten Teilen des Beutelsystems wird durch Öffnen oder Schließen der Absperrorgane 2, die als handelsübliche Schlauchklemmen ausgeführt sind, realisiert. Der Transport der flüssigen Medien, ggf. mit Lufteinschlüssen, wird im Beutelsystem unter Ausnutzung der Schwerkraft und durch manuelle Druckausübung auf die flexiblen Wände der Beutel realisiert. Das Einlaßorgan 4, welches bei dieser Ausführung ein Steril-Filter ist, dient dem sterilen Eintrag der Flüssigkeiten, die eine Kryokonservicrung ermöglichen und/oder unterstützen, d.h. insbesondere eine Flüssigkeit zur Vermeidung der Blutgerinnung (A), eine Flüssigkeit zur Verdünnung (B) und/oder ein flüssiges Kryoprotektikum (C).
Nach dem Eintrag des Nabelschnurblutes und/oder Flüssigkeiten, die eine Kryokonservierung ermöglichen und/oder unterstützen, können die Verbindungsleitungen 3.1 und 3.2, die aus einem PVC-weich-Material bestehen, durch Verschweißen, beispielsweise mit einem transportablen Folienschweißgerät, hermetisch verschlossen und/oder abgetrennt werden. Der flexible Misch- und Einfrier-Beutel 16 besteht ebenso, wie die Verbindungsleitungen 3, im wesentlichen aus einem EVA-Kunststoff-Material, Ethylenvinylacetat, das zumindest bis zu einer Temperatur von - 196 °C temperaturbeständig und durch eine bekannte Strahlensterilisation (GammaStrahlung) sterilisierbar ist. Auf dem transparenten Einfrier-Beutel 16, der für ein maximales Volumen von 180 ml ausgelegt ist, befindet sich eine Füllstandsanzeige 7, die die Werte für die Mindestfüllmenge von 60 ml und die Soll-Füllmenge von 160 ml anzeigt. Am Misch- und Einfrier-Beutel 16 ist ein Entnahmeadapter 13 angeordnet, der die sterile Entnahme des eingelagerten Flüssigkeitsgemisches ermöglicht. Die Verbindungsleitung 3.3 ist mit einem Auslassorgan 5, der ein Steril-Filter ist und insbesondere dem Luftaustrag aus dem Beutelsystem dient, verbunden. Die dünnwandige, flexible und transparente Verbindungsleitung 3.3 ist ein Bereich, zur Einlagerung des Gemisches aus Flüssigkeiten und der Körperflüssigkeit. Diese beispielsweise fünf Segmente 6 mit einem Fassungsvermögen von jeweils ca. 1 ml, die voneinander hermetisch verschließbar und abtrennbar sind, in Figur 1. nicht dargestellt, lassen sich durch Verschweißen, beispielsweise mit einem transportablen Folienschweißgerät, aus einem Teil der Verbiridungsleitung 3.3 herstellen.
Das Zusammenfügen der zuvor sterilisierten Teile des Beutelsystems, die aus handelsüblichen Teilen und Komponenten bestehen, zu einem geschlossenen System im Sinne der Erfindung erfolgt unter Reinstraumbedingungen. Für das Zusammenfügen sind für die Humanmedizin zugelassene Schweiß-, Klebe- und Fügetechniken anwendbar.

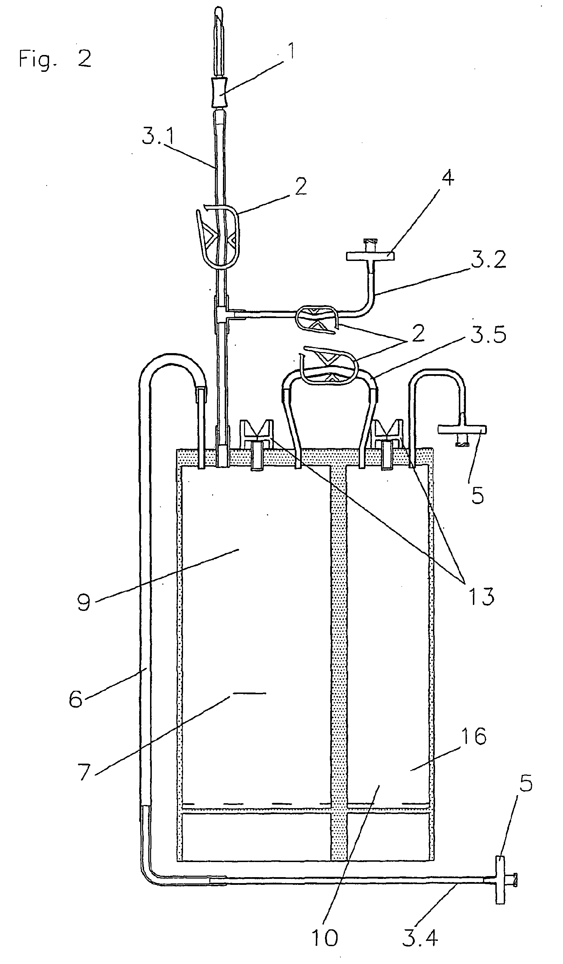

Das in Figur 2 dargestellte Beutelsystem zeigt einen Misch- und Einfrier-Beutel 16, der eine Misch- und Einfrier-Kammer 9 und eine Einfrierkammer 10 besitzt, die miteinander kommunizierend verbunden sind. Der Misch- und Einfrier-Beutel 16 kommuniziert über mehrere Verbindungsleitungen 3 mit einer Vorrichtung zur direkten Entnahme von Nabelschnurblut 1, einem Einlaßorgan 4 und zwei Auslassorganen 5.
Die beiden voneinander abtrennbaren Kammern, nämlich die Misch- und EinfrierKammer 9 und die Einfrierkammer 10 verfügen jeweils über einen Entnahmeadapter 13 und ein Auslassorgan 5.

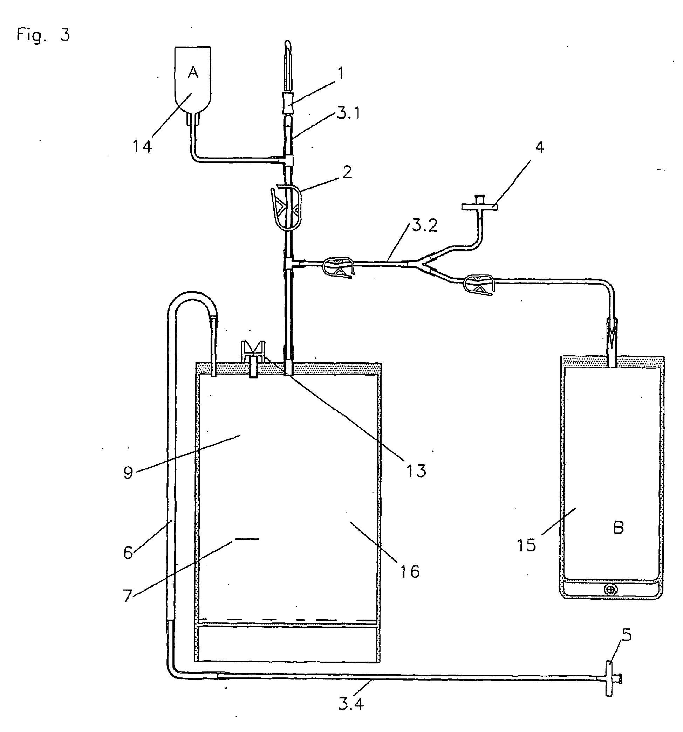

Die in Figur 3 aufgezeigte Ausführung unterscheidet sich von Figur 1 dadurch, dass ein Beutel 14, der 21 ml CPD enthält, einer Flüssigkeit zur Vermeidung der Blutgerinnung (A), und ein Beutel 15, der 100 ml einer Kochsalzlösung (NaCl) enthält, die jeweils über eine Verbindungsleitung 3 mit dem geschlossenen Beutelsystem verbunden sind, angeordnet sind. Die Beutel 14 und 15 besitzen jeweils hin zur Verbindungsleitung 3 ein Brechventil.

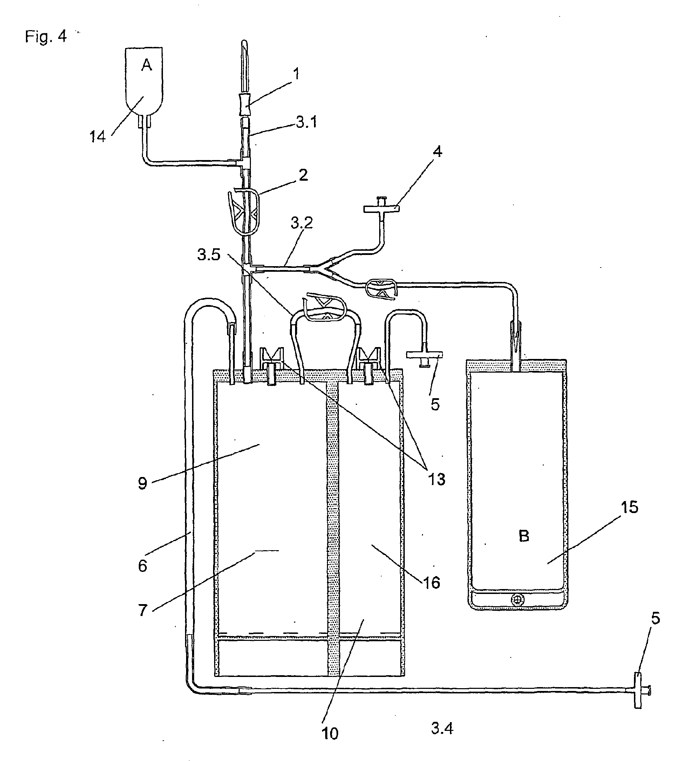

In Figur 4 besitzt das Beutelsystem einen Misch- und Einfrier-Beutel 16, der im Unterschied zu Figur 3 zwei Kammern enthält, nämlich eine Misch- und EinfrierKammer 9 und eine Einfrierkammer 10.

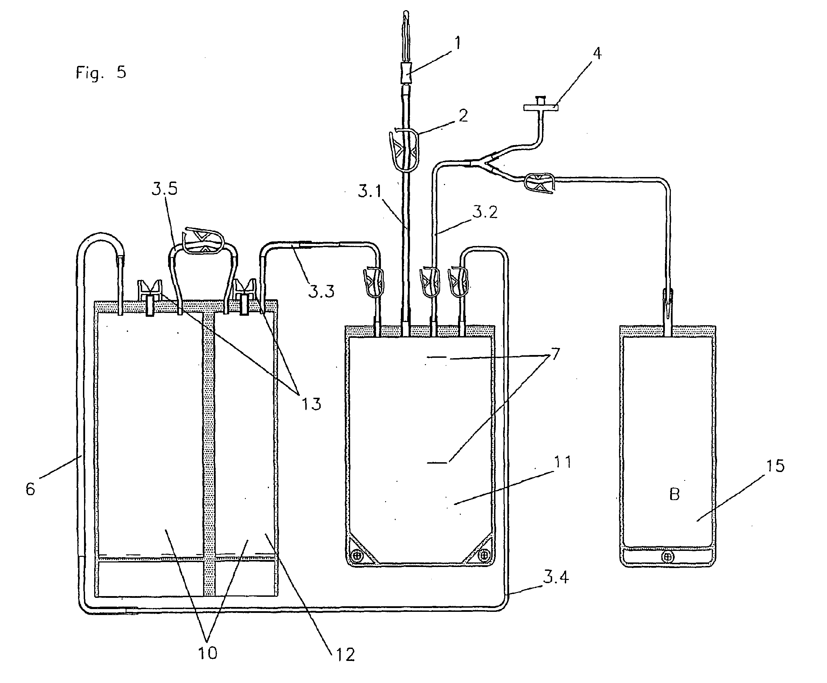

In Figur 5 ist ein Beutelsystem dargestellt, dass einen Misch-Beutel 11, der durch vier Verbindungsleitungen 3.1, 3.2, 3,3 und 3.4 mit einer Vorrichtung zur direkten Entnahme von Nabelschnurblut 1, einem Einlaßorgan 4, einem Einfrierbeutel 12 und einem Beutel 15 kommunizierend verbunden ist. Die Kommunkation, d.h. insbesondere der Flüssigkeitstransport und der Luftaustausch, zwischen den vorgenannten Teilen des Beutelsystems wird durch Öffnen oder Schließen der Absperrorgan 2 realisiert. Die flüssigen Medien, ggf. mit Lufteinschlüssen, werden im Beutelsystem unter Ausnutzung der Schwerkraft und durch manuelle Druckausübung auf die flexiblen Wände der Beutel gemischt, bewegt und transportiert. Das Einlaßorgan 4, welches bei dieser Ausführung ein Steril-Filter ist, dient dem sterilen Eintrag eines flüssigen Kryoprotektikum C, beispielsweise UMSO. Nach dem Eintrag des Nabelschnurblutes und/oder Flüssigkeiten, die eine Kryokonservierung ermöglichen und/oder unterstützen, können die Verbindungsleitungen 3.1 und 3.2 durch Verschweißen hermetisch verschlossen und/oder abgetrennt werden. Im Mischbeutel 11 kann CPD vorgelegt sein. Der flexible Misch-Beutel 11 besteht ebenso, wie die Verbindungsleitungen 3, im wesentlichen aus einem weich-PVC-Kunststoff-Material, das in an sich bekannter Art und Weise durch Autoklavierung sterilisierbar ist. Auf dem transparenten Misch-Beutel 11, der für ein maximales Volumen von 180 ml ausgelegt ist, befindet sich eine Füllstandsanzeige 7, die die Werte für die Mindestfüllmenge von 60 ml und die Soll-Füllmenge von 160 ml anzeigt. Am Einfrier-Beutel 12, der im wesentlichen aus einem EVA-Kunststoff-Material besteht und zwei Einfrierkammern 10 besitzt, ist an jeder Einfrierkammer 10 ein Entnahmeadapter 13 angeordnet, der die sterile Entnahme des eingelagerten Flüssigkeitsgemisches ermöglicht. Die Verbindungsleitungen 3.3 und 3.4 verbinden den Mischbeutel 11 und den Einfrierbeutel 12 miteinander. Eine der dünnwandigen, flexiblen und transparenten Verbindungsleitung 3.3 oder 3.4 kann ein Bereich zur Einlagerung des Gemisches aus Flüssigkeiten und der Körperflüssigkeit sein. Diese beispielsweise fünf Segmente 6 mit einem Fassungsvermögen von jeweils ca. 1 ml, die voneinander hermetisch verschließbar und abtrennbar sind, in Figur 5 nicht dargestellt, lassen sich durch Verschweißen, beispielsweise mit einem transportablen Folienschweißgerät, aus einem Teil der Verbindungsleitung 3.3 oder 3.4 herstellen. Die beiden Einfrierkammern 10 sind kommunizierend durch eine Verbindungsleitung 3.5 miteinander verbunden. Nach dem hermetischen Verschließen und Abtrennen der Verbindungsleitung 3.3, 3.4 und 3,5 vom Mischbeutel 11 und Einfrierbeutel 12, sind die beiden Einfrierkammern 10 voneinander abtrennbar.

Das Zusammenfügen der zuvor sterilisierten Teile des Beutelsystems, die aus handelsüblichen Teilen und Komponenten bestehen, zu einem geschlossenen System im Sinne der Erfindung erfolgt unter Remstraumbedingungen. Für das Zusammenfügen sind für die Humanmedizin zugelassene Schweiß-, Klebe-und Fügetechniken anwendbar.

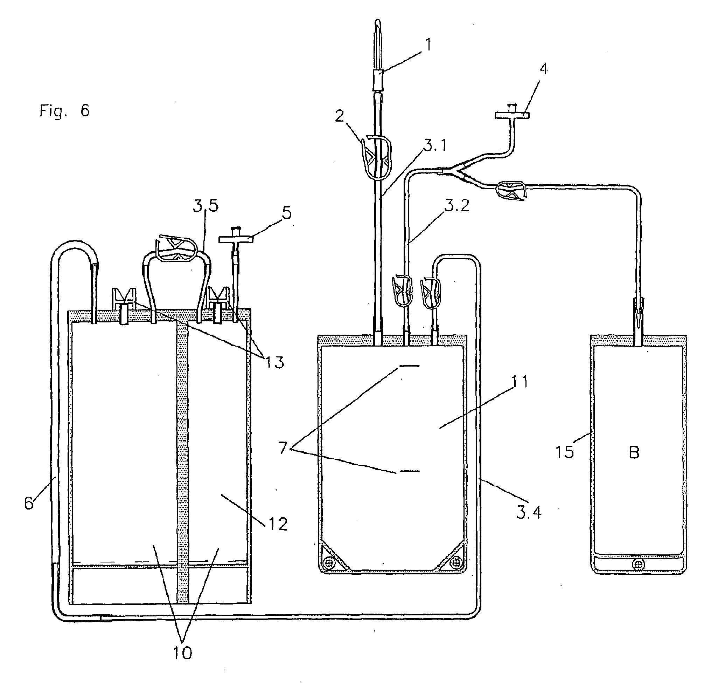

Figur 6 zeigt ein Beutelsystem analog Figur 5, wobei der Mischbeutel 11 und der Einfrierbeutel 12 durch eine Verbindungsleitung 3.4 verbunden sind. Die fehlende Verbindungsleitung 3.3 ist durch ein Auslassorgan 5, welches ein Steril-Filter ist, der insbesondere der Entlüftung dient, ersetzt.

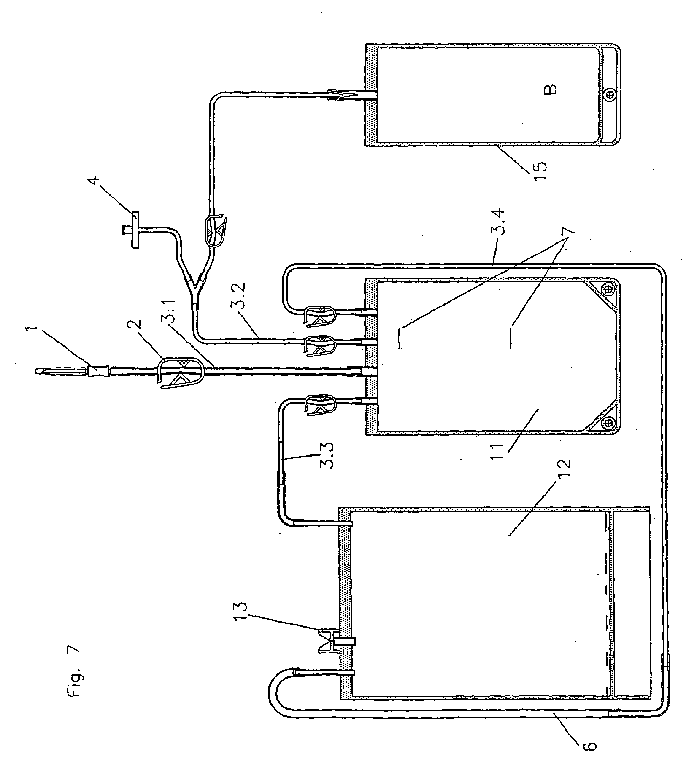

In Figur 7 ist das Beutelsystem analog Figur 5 dargestellt, wobei der Einfrierbeutel nur eine Einfrier-Kammer 10 besitzt.

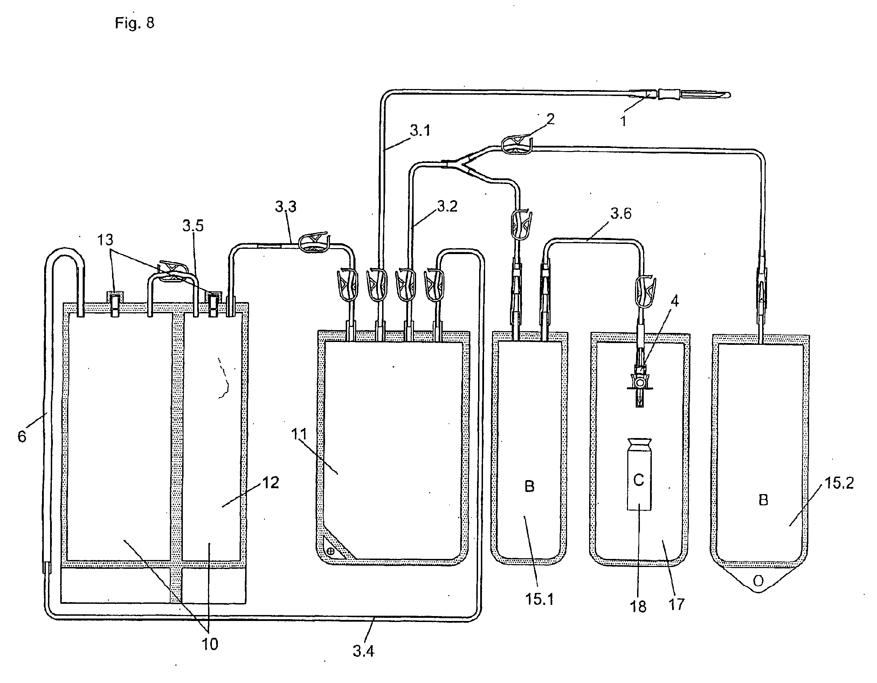

In Figur 8 ist ein Beutelsystem dargestellt, dass einen Misch-Beutel 11, der durch vier Verbindungsleitungen 3.1, 3.2, 3.3 und 3.4 mit einer Vorrichtung zur direkten Entnahme von Nabelschnurblut 1, einem Einfrierbeutel 12 und zwei Beuteln 15.1 und 15.2 kommunizierend verbunden ist. Die Kommunikation, d.h. insbesondere der Flüssigkeitstransport und der Luftaustausch, zwischen den vorgenannten Teilen des Beutelsystems wird durch Öffnen, oder Schließen der Absperrorgane 2 realisiert. Die flüssigen Medien, ggf. mit Lufteinschlüssen, werden im Beutelsystem unter Ausnutzung der Schwerkraft und durch manuelle Druckausübung auf die flexiblen Wände der Beutel transportiert. Das Einlaßorgan 4, welches bei dieser Ausführung ein Anstechdorn für einen Durchstechstopfen einer Glasampulle 18 ist, dient dem sterilen Eintrag eines flüssigen Kryoprotektikum C, beispielsweise 10 ml DMSO. Die Glasampulle 18 ist in einem hermetisch verschlossenen Schutzbeutel 17 angeordnet, der über eine Verbindungsleitung 3.6 mit dem Beutel 15.1 verbunden ist. Der Beutel 15.1, der ein Fassungsvermögen von ca. 20 ml besitzt, enthält eine Menge von ca. 1.0 ml einer Kochsalzlösung. Hin zu den Verbindungsleitungen 3.2 und 3.6 ist der Beutel 15.1 durch jeweils ein Brechventil verschlossen. Der Beutel 15.2, der über die Verbindungsleitung 3.2 mit dem Beutel 15.1 sowie dem Mischbeutel 11 verbunden und durch ein Brechventil verschlossen ist, enthält 100 mal einer Kochsalzlösung.
Nach dem Eintrag des Nabelschnurblutes und/oder Flüssigkeiten, die eine Kryokonservierung ermöglichen und/oder unterstützen, können die Verbindungaleitungen 3.1 und 3.2 durch Verschweißen hermetisch verschlossen und/oder abgetrennt werden. Der flexible Misch-Beutel 11 besteht ebenso, wie die Verbindungsleitungen 3, im wesentlichen aus einem weich-PVC-Kunststoff-Material, das in an sich bekannter Art und Weise durch Autoklavierung sterilisierbar ist. Der transparente Misch-Beutel 11 ist für ein maximales Volumen von 180 ml ausgelegt. Am Einfrier-Beutel 12, der im wesentlichen aus einem EVA-Kunststorff-Material besteht und zwei Einfrierkammern 10 besitzt, ist an jeder Einfrierkammern 10 ein Entnahmeadapter 13 angeordnet, der die sterile Entnahme des eingelagerten Flüssigheitsgemisches ermöglicht. Die Verbindungsleitungen 3.3 und 3.4 verbinden den Mischbeutel 11 und den Einfrierbeutel 12 miteinander. Eine der dünnwandigen, flexiblen und transparenten Verbindungsleitung 3.3 oder 3.4 kann ein Bereich zur Einlagerung des Gemisches aus Flüssigkeiten und der Körperflüssigkeit sein. Diese beispielsweise fünf Segmente 6 mit einem Fassungsvermögen von jeweils ca, 1 ml, die voneinander hermetisch verschließbar und abtrennbar sind, in Figur 8 nicht dargestellt, lassen sich durch Verschweißen aus einem Teil der Verbindungsleitung 3.3 oder 3.4 herstellen. Die beiden Einfrierkammern 10 sind kommunizierend durch eine Verbindungsleitung 3.5 miteinander verbunde. Nach dem hermetischen Verschließen und Abtrennen der Verbindungsleitung 3.3, 3.4 und 3.5 vom Mischbeutel 11 und Einfrierbeutel 12, sind die beiden Einfrierkammern 10 voneinander abtrennbar.

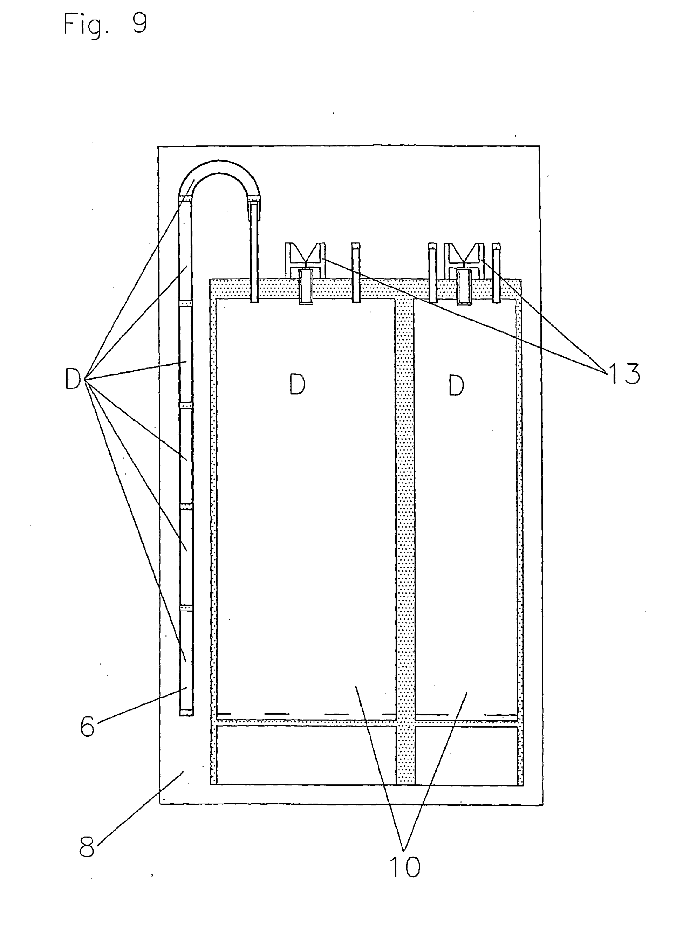

Figur 9 zeigt Teile eines Beutelsystemes vor dem Einfrieren. Nach dem Abtrennen der hermetisch verschlossenen Teile eines Beutelsystems, die der Kryokonservierung zugeführt werden sollen, nämlich eine oder zwei. Kammern des Einfrierbeutels 12 oder des Misch- und Einfrier-Beutels 16 und/oder die Segmente 6, die ein Flüssigkeitsgemisch D, bestehend aus Nabelschnurblut und Flüssigkeiten, die eine Kryokonservierung ermöglichen und/oder unterstützen, enthalten, erfolgt das Einbringen der vorgenannten Teile des Beutelsystems in eine Umhüllung 8. Diese Umhüllung 8, die insbesondere dem Schutz vor mechanischen Beschädigungen dient, besteht aus einer kältebeständigen Kunststoff-Folie, die durch Verschweißen gasdicht verschlossen ist.

### Bezugszeichenliste

Vorrichtung zur Entnahme der Körperflüssigkeit (1)
Absperrorgane (2)
Verbindungsleitungen (3)
Einlassorgan (4)
Auslassorgan (5)
Segmente (6)
Füllstandsanzeige (7)
Umhüllung (8)
Misch- und Einfrier-Kammer (9)
Einfrier-Kammer (10)
Mischbeutel (11)
Einfrierbeutel (12)
Entnahmeadapter (13)
Beutel mit A (14)
Beutel mit B (15)
Misch-und Einfrier-Beutel (16)
Schutzbeutel (17)
Glasampulle (18)
Flüssigkeit zur Vermeidung der Blutgerinnung (A)
Flüssigkeit zur Verdünnung (B)
Kryoprotektikum (C)
Flüssigkeitsgemisch (D)

## Patentansprüche

1. Beutelsystem für die Kryokonservierung von Körperflüssigkeiten, nämlich Blut, Knochenmark oder Nabelschnurblut, wobei die Teile des Beutelsystems sterilisiert, voneinander abtrennbar sind und sich Flüssigkeiten, die eine Kryokonservierung ermöglichen und/oder unterstützen, im Beutelsystem befinden und sich steril in das Beutelsystem eintragen lassen, zumindest umfassend:
- eine Vorrichtung zur direkten Entnahme der Körperflüssigkeit (1) aus dem lebenden Körper,
- zumindest einen Bereich zum Mischen der Flüssigkeiten mit der Körperflüssigkeit und zumindest einen Bereich zur Einlagerung des Gemisches aus Flüssigkeiten und Körperflüssigkeit, nämlich
einen Mischbeutel (11) und einen. Einfrierbeutel (12), wobei dieser Einfrierbeutel (12) eine Einfrierkammer oder zumindest zwei Einfrierkammern besitzt;
- Verbindungsleitungen (3) und mehrere Absperrorgane (2) zum Absperren jeweils einer der Verbindungsleitungen (3),
wobei die Teile des Beutelsystems, die durch die Verbindungsleitungen (3) miteinander kommunizierend verbunden sind, vor dem Einsatz des Beutelsystems ein geschlossenes, steriles System bilden,
und
die Bereiche zur Einlagerung des Gemisches aus Flüssigkeiten und der Körperflüssigkeit voneinander hermetisch verschließbar und abtrennbar sind, wobei das Beutelsystem ein Einlaßorgan (4) für Flüssigkeiten, welches ein Steril-Filter ist, umfasst, und der Mischbeutel (11) und der Einfrierbeutel (12) durch zwei Verbindungsleitungen (3) miteinander verbunden sind, wobei ein geschlossener Kreislauf zwischen den beiden Beuteln besteht.

2. Beutelsystem nach Anspruch 1, **dadurch gekennzeichnet, dass** ein Bereich zur Einlagerung des Gemisches aus Flüssigkeiten und der Körperflüssigkeit eine Verbindungsleitung (3) ist, die mehrere voneinander hermetisch verschließbare und abtrennbare Segmente (6) besitzt.

3. Beutelsystem nach Anspruch 1 bis 2, **dadurch gekennzeichnet, dass** zumindest im jeweiligen Bereich zum Mischen der Flüssigkeiten eine Füllstandsanzeige (7) angeordnet ist.

4. Beutelsystem nach Anspruch 1 bis 3, **dadurch gekennzeichnet, dass** nur die Bereiche, die zur Einlagerung des Gemisches aus Flüssigkeiten und der Körperflüssigkeit voneinander hermetisch verschließbar und abtrennbar sind, zur Kryokonservierung einlagerbar sind.

5. Beutelsystem nach Anspruch 1 bis 4, **dadurch gekennzeichnet, dass** die von den anderen Teilen des Systems abgetrennten Bereiche, die zur Einlagerung des Gemisches aus Flüssigkeiten und der Körperflüssigkeit dienen, von einer Umhüllung (8) luftdicht umschlossen ist.

6. Beutelsystem nach Anspruch 1 bis 5, **dadurch gekennzeichnet, dass** der Bereich zum Mischen der Flüssigkeiten mit der Körperflüssigkeit in einem Mischbeutel (11) und der Bereich zur Einlagerung des Gemisches aus Flüssigkeiten und Körperflüssigkeit in einem Einfrierbeutel (12) angeordnet sind, wobei dieser Beutel eine Einfrier-Kammer (9) oder zwei Einfrier-Kammern (9) besitzt.

## Claims

1. A bag system for the cryoconservation of body fluids, namely blood, bone marrow or umbilical cord blood, wherein the parts of the bag system can be sterilized or separated from each other, and wherein the bag system contains liquids which enable and/or support cryoconservation and which can be introduced into the bag system in a sterile mode, at least comprising:
- a device for direct taking the body fluid (1) off the living body;
- at least one compartment to mix the liquids with the body fluid, and at least one compartment to store the mixture of liquids and body fluid, namely
a mixing bag (11) and a freezing bag (12), said freezing bag (12) being provided with one freezing chamber or at least two freezing chambers;
- connecting lines (3) and several shut-off organs (2) to shut off any of the connecting lines (3) alone,
wherein the parts of the bag system which communicate with each other via the connecting lines (3) form a closed sterile system prior to the bag system being used, and
the compartments for storing the mixture of liquids and body fluid can be hermetically sealed off against and separated from each other, the bag system comprising an inlet organ (4) for liquids which is a sterile filter, and the mixing bag (11) and freezing bag (12) being connected by two connecting lines (3) in order to present a closed cycle between the two bags.

2. A bag system according to Claim 1, **characterized in that** one compartment to store the mixture of liquids and body fluid is a connecting line (3) which consists of several segments (6) which can be hermetically sealed off against and separated from each other.

3. A bag system according to any of Claims 1 or 2, **characterized in that** a filling level indicator is arranged in at least the compartment in which the liquids are mixed.

4. A bag system according to any of Claims 1 to 3, **characterized in that** only those compartments which serve for the storage of the mixture of liquids and the body fluid and which can be hermetically sealed off against and separated from each other, can be stored for the purpose of cryoconservation.

5. A bag system according to any of Claims 1 to 4, **characterized in that** the compartments separated from the other parts of the system and which serve for the storage of the mixture of liquids and the body fluid are enclosed in an airtight encapsulation (8).

6. A bag system according to any of Claims 1 to 5, **characterized in that** the compartment for the mixture of liquids and the body fluid is located in a mixing bag (11) and the compartment to store the mixture of liquids and the body fluid is located in a freezing bag (12), the latter being provided with one freezing chamber (9) or two freezing chambers (9).

## Revendications

1. Un système de sachets pour la cryopréservation de fluides corporels, à savoir du sang, de la moelle osseuse ou du sang du cordon ombilical, les composants du système de sachets étant stérilisés et qui peuvent être séparés entre eux, et le système de sachet contenant des liquides qui permettent et / ou soutiennent la cryopréservation et qui peuvent être introduits dans un mode stérile dans le système de sachets, comprenant au minimum :
- un dispositif qui permet l'enlèvement direct du fluide corporel (1) du corps vivant,
- au moins une zone pour mélanger les liquides avec les fluides corporels, et au moins une zone pour stocker le mélange de liquides et fluides corporels, à savoir
un sachet de mélange (11) et un sachet de congélation (12), ce dernier (12) comportant une chambre de congélation ou, au moins, deux chambres de congélation ;
- des lignes de connexion (3) et plusieurs organes d'arrêt (2) pour fermer l'une des lignes de connexion (3) en chaque fois,
les composants du système de sachets qui sont raccordés, communiquant ainsi, l'un à l'autre via les lignes de connexion (3), constituant, avant l'utilisation du système de sachets, un système clos stérile,
et
les zones où le mélange de liquides et fluides corporels peuvent être stocké, peuvent être fermés, et ainsi séparés, l'un contre l'autre hermétiquement, le système de sachets étant pourvu d'un organe d'admission (4) pour les liquides, qui est un filtre stérile, et le sachet de mélange (11) étant raccordé au sachet de congélation (12) par deux lignes de connexion (3), ainsi formant un cycle fermé entre les deux sachets.

2. Un système de sachets selon la revendication 1, **caractérisé en ce que** la zone où le mélange de liquides et fluides corporels peuvent être stocké, est une connexion (3), qui possède plusieurs segments (6) qui peuvent être fermés, et ainsi séparés, l'un contre l'autre hermétiquement.

3. Un système de sachets selon l'une des revendications 1 à 2, **caractérisé en ce que** la zone au moins qui sert chaque fois au mélange de liquides et fluides est pourvu d'un indicateur de niveau (7).

4. Un système de sachets selon l'une des revendications 1 à 3, **caractérisé en ce que** les seules zones qui peuvent être fermées, et ainsi séparées, l'une contre l'autre hermétiquement dans le but de stocker le mélange de liquides et fluides corporels, sont capable d'être stockées pour cryopréservation.

5. Un système de sachets selon l'une des revendications 1 à 4, **caractérisé en ce que** les zones qui sont séparées des autres composants du système et qui sont utilisées pour le stockage du mélange de liquides et fluides corporels, sont confinées par une enveloppe hermétique (8).

6. Un système de sachets selon l'une des revendications 1 à 5, **caractérisé en ce que** la zone qui sert au mélange des liquides avec les fluides corporels se trouve dans un sachet de mélange (11) et que la zone pour stocker le mélange de liquides et fluides corporels se trouve dans un sachet de congélation (12), ce dernier ayant pourvu d'une chambre de congélation (9) ou deux chambres de congélation (9).
